(19) Europäisches Patentamt
European Patent Office
Office européen des brevets

(11) Veröffentlichungsnummer : **0 252 350 B1**

# (12) EUROPÄISCHE PATENTSCHRIFT

(45) Veröffentlichungstag der Patentschrift : **02.01.91 Patentblatt 91/01**

(51) Int. Cl.$^5$ : **C07D 215/54**, // C09B29/36

(21) Anmeldenummer : **87108843.1**

(22) Anmeldetag : **20.06.87**

(54) **3-Cyanochinolinderivate.**

(30) Priorität : **21.06.86 DE 3620856**

(43) Veröffentlichungstag der Anmeldung : **13.01.88 Patentblatt 88/02**

(45) Bekanntmachung des Hinweises auf die Patenterteilung : **02.01.91 Patentblatt 91/01**

(84) Benannte Vertragsstaaten : **CH DE FR GB IT LI**

(56) Entgegenhaltungen :
**EP-A- 0 000 343**
**DE-A- 2 206 506**
**DE-A- 2 307 444**

(73) Patentinhaber : **BASF Aktiengesellschaft**
**Carl-Bosch-Strasse 38**
**D-6700 Ludwigshafen (DE)**

(72) Erfinder : **Hackenberger, Alfred, Dr.**
**Luitpoldstrasse 77**
**D-6700 Ludwigshafen (DE)**
Erfinder : **Patsch, Manfred, Dr.**
**Fritz-Wendel-Strasse 4**
**D-6706 Wachenheim (DE)**

EP 0 252 350 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Verbindungen der Formel I

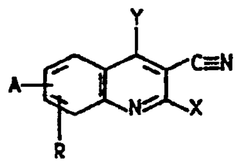

(I),

in der

A Nitro, Amino, Halogencarbonyl oder Halogensulfonyl,

R Wasserstoff, Halogen, Nitro, Cyano, $C_1$-$C_4$-Alkanoylamino, Hydroxy, $C_1$-$C_4$-Alkoxy, gegebenenfalls durch $C_1$-$C_4$-Alkyl, Halogen oder $C_1$-$C_4$-Alkoxy substituiertes Phenoxy, Mercapto, $C_1$-$C_4$-Alkylthio, gegebenenfalls durch $C_1$-$C_4$-Alkyl, Halogen oder $C_1$-$C_4$-Alkoxy substituiertes Phenylthio, Carboxyl, $C_1$-$C_4$-Alkoxycarbonyl, gegebenenfalls durch $C_1$-$C_4$-Alkyl, Halogen oder $C_1$-$C_4$-Alkoxy substituiertes Phenoxycarbonyl, Carbamoyl, Hydroxysulfonyl, $C_1$-$C_4$-Alkylsulfonyl, gegebenenfalls durch $C_1$-$C_4$-Alkyl, Halogen oder $C_1$-$C_4$-Alkoxy substituiertes Phenylsulfonyl, Sulfamoyl, Trifluormethyl oder gegebenenfalls durch Hydroxy, $C_1$-$C_4$-Alkoxy oder Halogen substituiertes $C_1$-$C_4$-Alkyl und

X und Y jeweils unabhängig voneinander Halogen, Hydroxysulfonyl, $C_1$-$C_4$-Alkoxy oder gegebenenfalls durch $C_1$-$C_4$-Alkyl, Halogen oder $C_1$-$C_4$-Alkoxy substituiertes Phenoxy bedeuten, mit der Maßgabe, daß mindestens einer der Reste X und Y für Halogen steht.

Für den Fall, daß die 3-Cyanochinolinderivate der Formel I einen oder mehrere Hydroxysulfonylreste und/oder einen Carboxylrest aufweisen, sollen auch deren Salze, insbesondere die Alkalisalze, z.B. die Natrium- oder Kaliumsalze, mit umfaßt sein.

Für den Fall, daß die in Formel I auftretenden Phenylreste durch Halogen substituiert sind, kommen als Substituenten insbesondere Fluor, Chlor oder Brom in Betracht.

R, X und Y in Formel I bedeuten beispielsweise Fluor, Chlor, Brom, Methoxy, Ethoxy, Propoxy, Isopropoxy, Butoxy, Isobutoxy, sec-Butoxy, Phenoxy, 4-Methylphenoxy, 2-Chlorphenoxy, 2,4-Dichlorphenoxy oder 4-Methylphenoxy.

R in Formel I bedeutet weiterhin z.B. Formylamino, Acetylamino, Propionylamino, Butyrylamino, Isobutyrylamino, Methylthio, Ethylthio, Propylthio, Isopropylthio, Butylthio, Isobutylthio, sec-Butylthio, Phenylthio, 2-Ethylphenylthio, 4-Fluorphenylthio, 4-Methoxyphenylthio, Methoxycarbonyl, Ethoxycarbonyl, Propoxycarbonyl, Butoxycarbonyl, sec-Butoxycarbonyl, Phenoxycarbonyl, 4-Isopropylphenoxycarbonyl, 2-Bromphenoxycarbonyl, 4-Methoxyphenoxycarbonyl, Methylsulfonyl, Ethylsulfonyl, Propylsulfonyl, Butylsulfonyl, 2-Ethylphenylsulfonyl, 2,4-Dichlorphenylsulfonyl, 4-Isopropoxyphenylsulfonyl, Methyl, Ethyl, Propyl, Isopropyl, Butyl, Isobutyl, sec-Butyl, 2-Chlorethyl, 2-Hydroxyethyl, 2-Methoxyethyl, 2-Ethoxyethyl oder 2- oder 3-Hydroxypropyl.

Bevorzugt sind Cyanochinoline der Formel I, in der X und Y Fluor bedeuten, oder einer der Reste X und Y für Fluor oder Chlor und der andere für Hydroxysulfonyl steht.

Von besonderer Bedeutung sind Verbindungen der Formel II

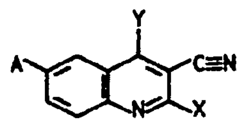

II,

in der A Nitro oder Amino und X und Y Fluor, Chlor oder Hydroxysulfonyl bedeuten.

Zur Herstellung der erfindungsgemäßen Verbindungen kann man beispielsweise ein Isatosäureanhydrid der Formel III

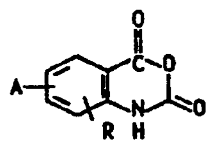

(III),

in der A und R jeweils die obengenannte Bedeutung besitzen, mit Cyanessigsäurealkylester umsetzen. Das resultierende 3-Cyano-2,4-dihydroxychinolinderivat kann anschließend nach an sich bekannten Methoden, z.B. durch Reaktion mit Phosphoroxychlorid in die entsprechende 2,4-Dichlorverbindung übergeführt werden.

Die Dichlorverbindungen können dann mit Metallfluoriden, z.B. Natrium- oder Kaliumfluorid, oder mit Fluorwasserstoff in die entsprechenden 2,4-Difluorverbindungen übergeführt werden.

Aus den Dichlor- und den Difluorverbindungen können durch eine nucleophile Substitutionsreaktion, z.B. mit dem Sulfitanion oder mit Hydroxyverbindungen, wie Methanol, Ethanol oder Phenol, solche erfindungsgemäßen Verbindungen, bei denen ein Halogenatom ausgetauscht ist, erhalten werden.

Die Einführung einiger der Substituenten A und R kann auch direkt am 3-Cyano-2,4-dihydroxychinolin durch elektrophile Substitution erfolgen. Beispielhalft sei die Nitrierung oder Sulfonierung genannt.

Die erfindungsgemäßen Verbindungen eignen sich als Anker in Reaktivfarbstoffen, als Vorprodukte für Wirkstoffe und für den Fall, daß A eine Aminogruppe bedeutet auch als Diazokomponenten.

Die folgenden Beispiele sollen die Erfindung näher erläutern. In ihnen beziehen sich die Angaben

über Teile und Prozente, sofern nicht anders vermekt, auf das Gewicht.

Beispiel 1

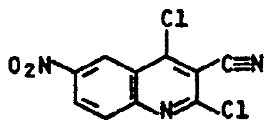

3-Cyano-2,4-dihydroxy-6-nitrochinolin

93 Teile 3-Cyano-2,4-dihydroxychinolin wurden in 990 Teilen konzentrierter Schwefelsäure gelöst. Bei einer Temperatur von 0-10°C wurde eine Mischung aus 212 Teilen konzentrierter Schwefelsäure und 161 Teilen 65%iger Salpetersäure zugetropft. Man ließ 2 Stunden nachrühren, fällte auf Eis, saugte das ausfallende Nitrochinolin ab und wusch mit Wasser säurefrei. Nach dem Trocknen erhielt man 102 Teile Rohprodukt (entsprechend einer Ausbeute von 88,3%).

3-Cyano-2,4-dichlor-6-nitrochinolin

In 770 Teile Phosphoroxychlorid wurden 231 Teile 3-Cyano-2,4-dihydroxy-6-nitrochinolin eingetragen. Danach wurden 109 Teile Triethylamin so zugetropft, daß die Temperatur auf 80°C anstieg. Nach einer Stunde Nachrühren wurden weitere 231 Teile 3-Cyano-2,4-dihydroxy-6-nitrochinolin eingetragen und nochmals 109 Teile Triethylamin zugetropft. Die Temperatur wurde danach langsam auf 140°C erhöht. Man ließ 2 Stunden bei dieser Temperatur nachrühren, kühlte ab, goß auf Eiswasser, saugte ab und wusch mit Wasser säurefrei. Nach dem Trocknen erhielt man 505 Teile Rohprodukt von 3-Cyano-2,4-dichlor-6-nitrochinolin (entsprechend einer Ausbeute von 94%). Eine aus Essigester umkristallisierte Probe hat einen Schmelzpunkt von 190 - 192°C.

Beispiel 2

3-Cyano-2,4-difluor-6-nitrochinolin

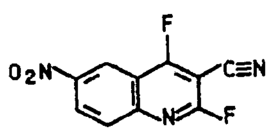

107 Teile 3-Cyano-2,4-dichlor-6-nitrochinolin und 69 Teile KF wurden in 520 Teilen Toluol suspendiert. Man destillierte das Toluol ab und gab 480 Teile N,N-Dimethylformamid zu. Danach ließ man 3 Stunden bei 90°C rühren, kühlte ab, goß auf Eiswasser, saugte das ausgefallene Produkt ab und wusch mit Wasser salzfrei. Nach der Trocknung erhielt man 90 Teile 3-Cyano-2,4-difluor-6-nitrochinolin (entsprechend einer Ausbeute von 96%). Eine aus Essigester umkristallisierte Probe hat einen Schmelzpunkt von 128 - 129°C.

Beispiel 3

6-Amino-3-cyano-2,4-difluorchinolin

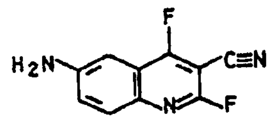

130 Teile wasserfreies Natriumsulfat wurden in einer Lösung aus 305 Teilen 3-Cyano-2,4-difluor-6-nitrochinolin in 3400 Teilen Tetrahydrofuran suspendiert. Man fügt 30 Teile eines Hydrierkatalysators (10% Palladium auf Kohle) zu und hydrierte bei einer Temperatur von 40°C und einem Druck von 3 bar. Nach Beendigung der Wasserstoffaufnahme wurde die Suspension filtriert und die klare Lösung zu 6000 Teilen Eiswasser gegeben. Der entstandene Niederschlag wurde abgesaugt, gewaschen und getrocknet. Man erhielt 198 Teile 6-Amino-3-cyano-2,5-difluorchinolin.Dies entspricht einer Ausbeute von 74%.

Das Produkt hat einen Schmelzpunkt von 199 - 202°C, wobei Zersetzung unter Gasentwicklung eintritt.

Beispiel 4

6-Amino-3-cyano-2,4-dichlorchinolin

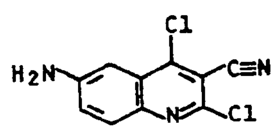

Man erhielt die Verbindung durch Reduktion von 3-Cyano-2,4-dichlor-6-nitrochinolin analog Beispiel 3. UV (THF) : $\lambda_{max}/\varepsilon$ = 415,2 (3189), 304,6 (7675), 274,0 (43425).

Beispiel 5

Umsetzung von 6-Amino-3-cyan-2,4-difluorchinolin mit Natriumsulfit

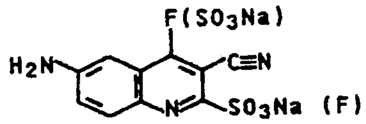

79 Teile wasserfreies Natriumsulfit wurden in 500 Teilen Wasser gelöst. Man gibt 82 Teile 6-Amino-3-cyano-2,4-difluorchinolin zu und ließ 3 Stunden nachrühren. Man kühlte auf 10°C ab, saugte das ausgefallene Produkt ab und wusch mit wenig Wasser nach. Nach dem Trocknen erhielt man 112 g eines einheitlichen Produktes, in dem ein Fluoratom gegen die Sulfonsäuregruppe ausgetauscht ist. Die Sulfonsäure fiel dabei als Natriumsalz mit einem Molekül Kristallwasser an. Die Ausbeute beträgt 90%.

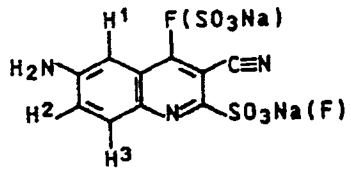

$^1$H-NMR ($d_6$.DMSO) : $\delta$ = 8,02 (d,1H,$H^1$) ; 7,80 - 7,31 (s,s,d,d,2H, AB-System$H^2H^3$ ; $J_{AB}$ = 9Hz), 5,95 (s,2H,$NH_2$), 3,43 (s,2H,$H_2O$) ; UV ($H_2O$) : $\lambda_{max}/\varepsilon$ = 407,7 (3057), 265 (27744) $\varepsilon$ ist dabei auf wasserfreies Na-Salz bezogen.

Beispiel 6

Umsetzung von
6-Amino-3-cyano-2,4-dichlorchinolin mit
Natriumsulfit

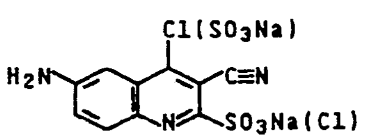

118 Teile wasserfreies Natriumsulfit wurden in 500 Teilen Wasser gelöst. Man gab 95 Teile 6-Amino-3-cyano-2,4-dichlorchinolin zu, rührte 3 Stunden bei 80°C nach, kühlte auf 10°C ab, saugte das ausgefallene Produkt ab und wusch mit wenig Wasser nach. Nach der Trocknung erhielt man 83 g eines einheitlichen Produktes, in dem ein Chloratom gegen die Sulfonsäuregruppe ausgetauscht ist. Die Sulfonsäure fiel dabei als Natriumsalz mit einem Molekül Kristallwasser an.

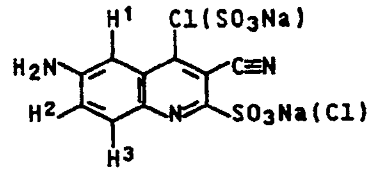

$^1$H-NMR ($d_6$.DMSO) : $\delta$ = 8,02 (d,1H,$H^1$) ;7,85 - 7,30 (s,s,d,d,2H, AB-System$H^2H^3$ ; $J_{AB}$ = 9Hz), 6,10 (s,2H,$NH_2$), 3,40 (s,2H,$H_2O$) ; UV ($H_2O$) : $\lambda_{max}/\varepsilon$ = 417,2 (2978), 275 (321111) $\varepsilon$ ist dabei auf wasserfreies Na-Salz bezogen.

Beispiel 7

Umsetzung von 3-Cyano-2,4-difluor-6-nitrochinolin mit Ethanol

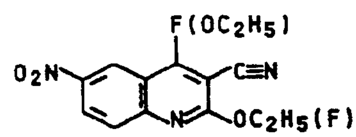

188 Teile 3-Cyano-2,4-difluor-6-nitrochinolin wurden in 1200 Teilen abs. Ethanol gelöst. Man suspendierte 51 Teile Natriumcarbonat in der Lösung und kochte zwei Stunden unter Rückfluß. Danach wurde heiß filtriert, das Filtrat auf 0 - 5°C abgekühlt und die dabei ausgefallenen Kristalle abgesaugt.

Man wusch mit wenig Ethanol nach und erhielt nach Trocknung 117 Teile eines einheitlichen Produktes, in dem ein Fluoratom gegen die Ethoxygruppe ausgetauscht ist. Die Verbindung hat einen Schmelzpunkt von 130 - 132°C.

Beispiel 8

Die Umsetzung von 3-Cyano-2,4-dichlor-6-nitrochinolin mit Ethanol analog Beispiel 7 lieferte eine Verbindung der folgenden Formel (Schmelzpunkt) 176 - 178°C).

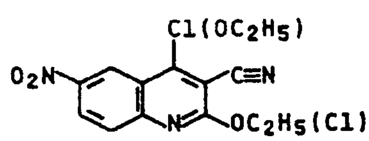

Beispiel 9

Durch katalytische Reduktion des in Beispiel 7 erhaltenen Produktes in Tetrahydrofuran (Katalysator

Pd/C, 10%) erhielt man ein Amin der folgenden Formel (Zersetzungspunkt 178°C).

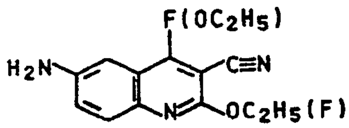

Beispiel 10

6-Chlorsulfonyl-3-cyano-2,4-dichlorchinolin

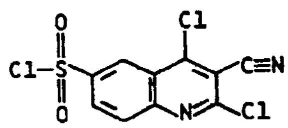

a) 3-Cyano-2,4-dihydroxy-6-hydroxysulfonylchinolin

In 1000 Teile Chlorsulfonsäure wurden 280 Teile 3-Cyano-2,4-dihydroxychinolin eingetragen. Man ließ 5 Stunden bei 80°C rühren, kühlte ab, goß auf Eiswasser, saugte ab und wusch mit Wasser nach. Das nach Trocknung erhaltene Rohprodukt kann ohne Reinigung zur Chlorierung eingesetzt werden.

b) 6-Chlorsulfonyl-3-cyano-2,4-dichlorchinolin

Das unter a) erhaltene Produkt wurde analog Beispiel 1 chloriert. Man erhielt 6-Chlorsulfonyl-3-cyano-2,4-dichlorchinolin, das nach Umkristallisieren aus Essigester einen Schmelzpunkt von 168 - 171°C hat.

**Ansprüche**

1. Verbindungen der Formel I

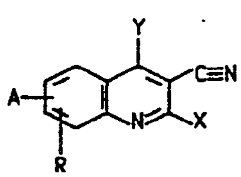

(I),

in der

A Nitro, Amino, Halogencarbonyl oder Halogensulfonyl,

R Wasserstoff, Halogen, Nitro, Cyano, $C_1$-$C_4$-Alkanoylamino, Hydroxy, $C_1$-$C_4$-Alkoxy, gegebenenfalls durch $C_1$-$C_4$-Alkyl, Halogen oder $C_1$-$C_4$Alkoxy substituiertes Phenoxy, Mercapto, $C_1$-$C_4$-Alkylthio, gegebenenfalls durch $C_1$-$C_4$-Alkyl, Halogen oder $C_1$-$C_4$ Alkoxy substituiertes Phenylthio, Carboxyl, $C_1$-$C_4$-Alkoxycarbonyl, gegebenenfalls durch $C_1C_4$-Alkyl, Halogen oder $C_1$-$C_4$-Alkoxy substituiertes Phenoxycarbonyl, Carbamoyl, Hydroxysulfonyl, $C_1$-$C_4$-Alkylsulfonyl, gegebenenfalls durch $C_1$-$C_4$-Alkyl, Halogen oder $C_1$-$C_4$-Alkoxy substituiertes Phenylsulfonyl, Sulfamoyl, Trifluormethyl oder gegebenenfalls durch Hydroxy, $C_1$-$C_4$ Alkoxy oder Halogen substituiertes $C_1$-$C_2$-Alkyl und

X und Y jeweils unabhängig voneinander Halogen, Hydroxysulfonyl, $C_1$-$C_4$-Alkoxy oder gegebenenfalls durch $C_1$-$C_4$-Alkyl, Halogen oder $C_1$-$C_4$-Alkoxy substituiertes Phenoxy bedeuten, mit der Maßgabe, daß mindestens einer der Reste X und Y für Halogen steht.

2. Verbindungen gemäß Anspruch 1, die die Formel II

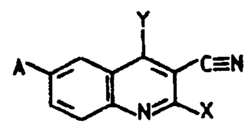

(II)

aufweisen,
in der A Nitro oder Amino und X and Y jeweils unabhängig voneinander Fluor, Chlor oder Hydroxysulfonyl bedeuten.

3. Verwendung der Verbindungen gemäß Anspruch 1 als Reaktivanker oder als Diazokomponenten.

**Claims**

1. A compound of the formula I

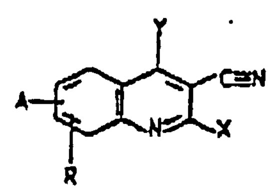

(I)

where

A is nitro, amino, halocarbonyl or halosulfonyl,

R is hydrogen, halogen, nitro, cyano, $C_1$-$C_4$-alkanoyl-amino, hydroxyl, $C_1$-$C_4$-alkoxy unsubstituted or $C_1$-$C_4$-alkyl-, halogen- or $C_1$-$C_4$-alkoxy-substituted phenoxy, mercapto, $C_1$-$C_4$-alkylthio, unsubstituted or $C_1$-$C_4$-alkyl-, halogen- or $C_1$-$C_4$-alkoxy-substituted phenylthio, carboxyl, $C_1$-$C_4$-alkoxycarbonyl, unsubstituted or $C_1$-$C_4$-alkyl-, halogen- or $C_1$-$C_4$-alkoxy-substituted phenoxycarbonyl, carbamoyl, hydroxysulfonyl, $C_1$-$C_4$-alkylsulfonyl, unsubstituted or $C_1$-$C_4$-alkyl-, halogen- or $C_1$-$C_4$-alkoxy-substituted phenyl-sulfonyl, sulfamoyl or trifluoromethyl or is unsubstituted or hydroxyl-, $C_1$-$C_4$-alkoxy- or halogen-substituted $C_1$-$C_4$al-

kyl, and

X and Y are each, independently of one another, halogen, hydroxysulfonyl, $C_1$-$C_4$-alkoxy or unsubstituted or $C_1$-$C_4$-alkyl-, halogen- or $C_1$-$C_4$-alkoxysubstituted phenoxy, subject to the proviso that at least one of X and Y is halogen.

2. A compound as claimed in claim 1, of the formula II

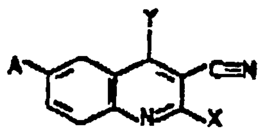

(II)

where

A is nitro or amino and X and Y are each independently of one another fluorine, chlorine or hydroxysulfonyl.

3. The use of a compound as claimed in claim 1 as a fiber-bonding site of a reactive dye or as a diazo component.

## Revendications

1. Composés de formule I

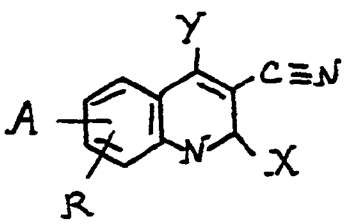

dans laquelle

A est un groupement nitro, amino, halogénocarbonyle ou halogénosulfonyle,

R est un atome d'hydrogène ou d'halogène ou un groupement nitro, cyano, alcanoylamino en $C_1$-$C_4$, hydroxy, alcoxy en $C_1$-$C_4$ ; phénoxy éventuellement substitué par des radicaux alkyle en $C_1$-$C_4$, halogéno ou alcoxy en $C_1$-$C_4$, mercapto, alkyltio en $C_1$-$C_4$ ; phénylthio éventuellement substitué par des radicaux alkyle en $C_1$-$C_4$, halogéno ou alcoxy en $C_1$-$C_4$ ; carboxyle, (alcoxy en $C_1$-$C_4$)carbonyle ; phénoxycarbonyle éventuellement substitué par des radicaux alkyle en $C_1$-$C_4$, halogèno ou alcoxy en $C_1$-$C_4$ ; carbamoyle, hydroxysulfonyle, alkylsulfonyle en $C_1$-$C_4$ ; phénylsulfonyle éventuellement substitué par des radicaux alkyle en $C_1$-$C_4$, halogèno ou alcoxy en $C_1$-$C_4$ ; sulfamoyle, trifluorométhyle ou alkyle en $C_1$-$C_4$ éventuellement substitué par des radicaux hydroxy, alcoxy en $C_1$-$C_4$ ou halogèno, et

X et Y sont indépendamment l'un de l'autre un atome d'halogène ou un groupement hydroxysulfonyle, alcoxy en $C_1$-$C_4$ ou phénoxy, ce dernier éventuellement substitué par des radicaux alkyle en $C_1$-$C_4$, halogèno ou alcoxy en $C_1$-$C_4$, pourvu que l'un au moins des restes X et Y soit un atome d'halogène.

2. Composés selon la revendication 1, qui présentent la formule II

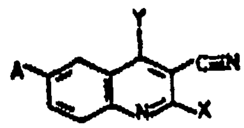

.(II)

dans laquelle A représente un groupement nitro ou amino et X et Y représentent indépendamment l'un de l'autre un atome de fluor ou de chlore ou un groupement hydroxysulfonyle.

3. Utilisation de composés selon la revendication 1, en tant qu'armatures de réactif ou en tant que composants de diazoïque.